# EUROPEAN PATENT SPECIFICATION

(11) **EP 1 256 331 B1**
(45) Date of publication and mention of the grant of the patent: **01.05.2013**
(21) Application number: 02253158.6
(22) Date of filing: 03.05.2002
(51) Int. Cl.: A61F 13/534

(54) **Body fluid absorbent article**
Absorbierender Artikel für Körperflüssigkeiten
Article absorbant les fluides corporels

(30) Priority: 07.05.2001 JP 2001136404
(43) Date of publication of application: 13.11.2002
(73) Proprietor: UNI-CHARM CORPORATION, Shikokuchuo-shi, Ehime-ken (JP)
(72) Inventor: Ishikawa, Norihiko, Technical Center, Mitoyo-gun, Kagawa-ken 769-1602 (JP); Inoue, Toshio, Technical Center, Mitoyo-gun, Kagawa-ken 769-1602 (JP); Abe, Kozo, Technical Center, Mitoyo-gun, Kagawa-ken 769-1602 (JP)
(74) Representative: Eke, Philippa Dianne

(56) References cited:
- EP-A- 0 425 269
- US-A- 4 235 237
- US-A- 6 140 550

## Description

This invention relates to body fluid absorbent articles such as disposable diapers, menstruation pads and the like for absorption and containment of body fluids.

Japanese Patent Application No. 1996-196559A discloses a disposable diaper having a liquid-absorbent core disposed between a liquid-pervious topsheet and a liquid-impervious backsheet wherein the liquid-absorbent core comprises a fibrous assembly layer placed upon a liquid-retaining layer formed by a mixture of fluff pulp and super-absorbent polymer particles.

The liquid-retaining layer and the fibrous assembly layer of the core are intermittently joined together substantially over the entire surfaces thereof opposed to each other and these two layers are integrally covered with tissue paper. These two layers and the tissue paper integrally covering them are also intermittently joined together substantially over the entire surfaces thereof opposed to each other. This diaper is claimed to be able to maintain the liquid-retaining layer, the fibrous assembly layer and the tissue paper in close contact with one another even when the diaper is bent or twisted. It is also claimed that the liquid-retaining layer is prevented from losing its shape even though this layer is less flexible and apt to get loose due to a large quantity of super-absorbent polymer particles contained therein.

With the diaper disclosed in the above-cited Publication, if the tissue paper is torn during use of the diaper, the liquid-retaining layer and the tissue paper may be peeled off from each other and the diaper may not keep its original shape. Once having been out of shape, the liquid-retaining layer can not restore its initial shape. In order that the core may achieves its proper function, the core is preferably formed by the liquid-retaining layer alone. However, in the case of the diaper disclosed by the above-cited Publication, the liquid-retaining layer itself has no means adapted to prevent this layer from losing its original shape.

EP 0 425 269 A2 discloses a melt spun fiber blend of a conventional synthetic material and a super-absorbent polymer and a process for its production. The fibers are useful in wound dressings, athletic clothing and other uses where high water absorbance of a fibre is important.

US 6,140,550 discloses an absorbent article including a flexible, fibrous support structure in a fixed shaped having particles of a super-absorbent material adhered thereto. Sufficient spacing is maintained between adjacent super-absorbent particles such that liquid can more freely enter the absorbent article for contact with the super-absorbent particles.

WO 01/06047 A1 discloses a method for manufacturing a yarn containing super-absorbent fibers of polyacrylate, wherein these fibers are blended with supporting fibers of a material stronger than that of the super-absorbent fibers.

The present invention provides the body fluid absorbent article of independent Claim 1. The dependent claims specify preferred but optional features.

The absorbent material according to this invention will not lose its shape and does not require other members than the absorbent material itself to keep the shape of the absorbent material. This is due to the reason that the absorbent material is a woven fabric made of yarns and each of the yarns functions to restricts relative movement of the yarns.

Movement of the disposable diaper, the sanitary napkin or the like using the article of this invention is not restricted by the article because the absorbent material can be freely deformed to follow the deformation of the diaper or the napkin. In the diaper or the napkin using the absorbent material of this invention, body fluids can be reliably absorbed and contained by the absorbent material, so there is no anxiety that body fluids might flow back to the topsheet and stick to the wearer's skin.
Fig. 1 is a perspective view showing a disposable diaper as viewed from the side of a topsheet and partially cutaway;
Fig. 2 is a perspective view of an absorbent material;
Fig. 3 is a perspective view showing a yarn constituting the absorbent material; and
Fig. 4 is an enlarged perspective view of a single synthetic fiber;

Details of a body fluid absorbent article according to this invention will be more fully understood from the description of an open-type disposable diaper and a sanitary napkin using the article of this invention, with reference to the accompanying drawings.

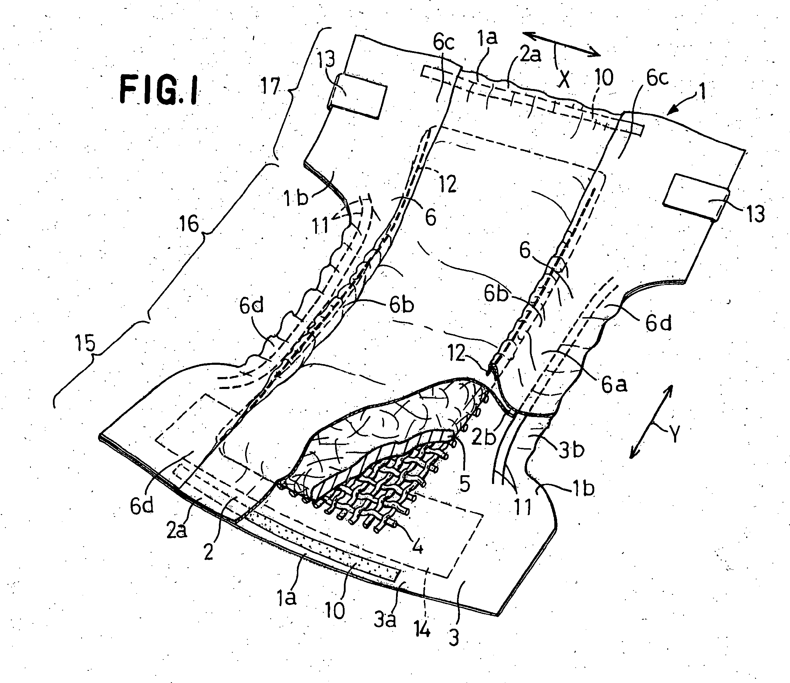

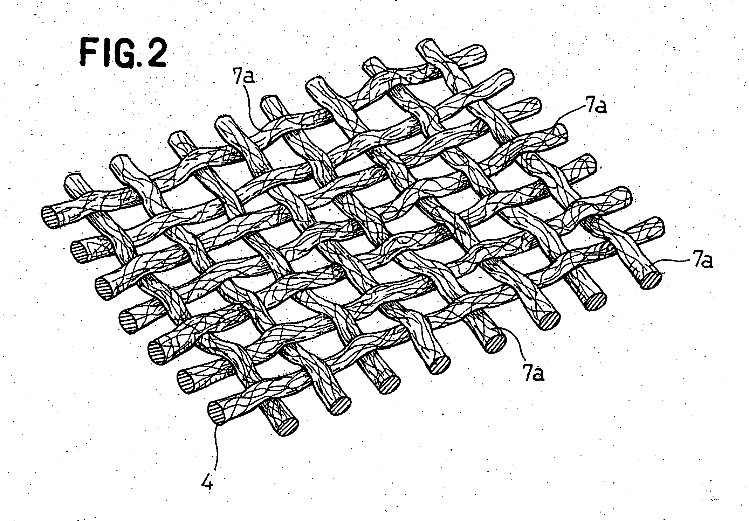

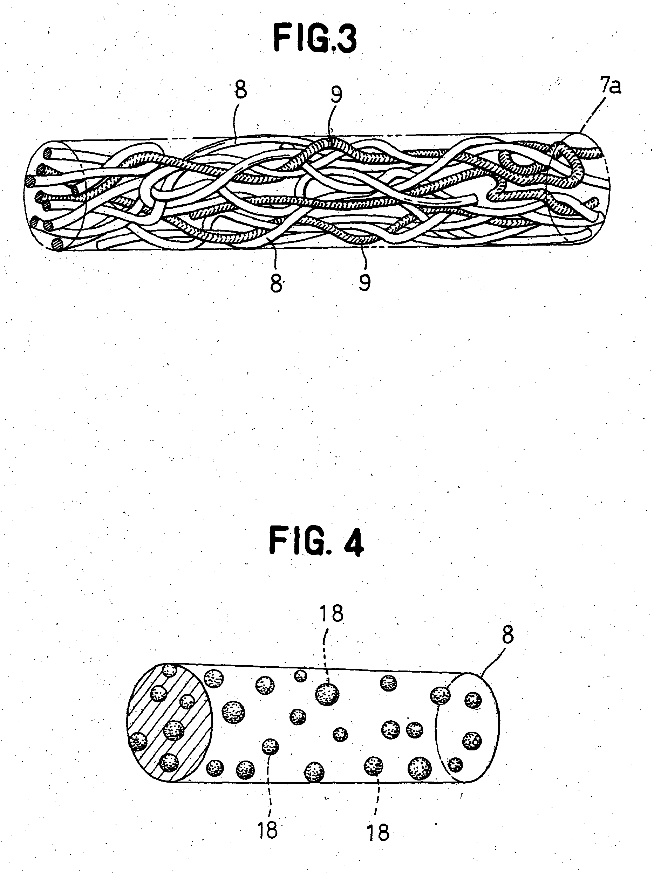

Fig. 1 is a perspective view showing a disposable diaper 1 as viewed from the side of a topsheet 2 and partially cutaway, Fig. 2 is a perspective view showing an absorbent material 4, Fig. 3 is a perspective view showing a yarn 7a constituting the absorbent material 4 and Fig. 4 is an enlarged perspective view of a synthetic fiber 8. In Fig. 1, a transverse direction of the diaper 1 is indicated by an arrow X and its longitudinal direction is indicated by an arrow Y. An expression 'inner surfaces' of topsheet 2 and backsheet 3, respectively, used herein should be understood to be the surfaces facing the absorbent material 4 and an expression 'outer surfaces' of these sheets 2, 3, respectively, should be understood to be the surfaces facing away from the absorbent material 4.

The diaper 1 comprises the liquid-pervious topsheet 2 facing a wearer's skin, the liquid-impervious backsheet 3 facing away from the wearer's skin, and the absorbent material 4 and a fibrous assembly 5 both being disposed between the top-and backsheets 2, 3. The fibrous assembly 5 is placed upon the upper side of the absorbent material 4. The diaper 1 further comprises substantially liquid-impervious leak-barrier cuffs 6 lying on the outer side of the topsheet 2 and extending in the longitudinal direction.

The diaper 1 is composed, as viewed in the longitudinal direction, of a front waist region 15, a rear waist region 17 and a crotch region 16 extending between these two waist regions 15, 17 and contoured by longitudinally opposite ends 1a extending across the front and rear waist regions 15, 17 in the transverse direction and transversely opposite side edges 1b extending in the longitudinal direction. In the crotch region 16, the transversely opposite side edges 1b describe circular arcs which are convex inwardly in the transverse direction of the diaper 1.

The absorbent material 4 is joined to the inner surface of the backsheet 3. The fibrous assembly 5 is joined to the inner surface of the topsheet 2. The absorbent material 4 and the fibrous assembly 5 are joined together along the surfaces thereof opposed to each other.

The absorbent material 4 is a net-like woven fabric woven by the yarns 7a comprising synthetic fibers 8 and regenerated fibers 9. As will be apparent from Fig. 4, within the synthetic fibers 8, super-absorbent polymer particles 18 are included. such synthetic fibers 8 may be obtained by spinning a molten thermoplastic synthetic resin blended with the super-absorbent polymer particles 18.

The synthetic fibers 8 are made of polyolefine-based fibers. As for the polyolefine-based fibers, at least one of polypropylene-based fibers and polyethylene-based fibers may be used. It is also possible to use core-sheath type conjugated fibers, side-by-side type conjugated fibers or sea-island type conjugated fibers of polyethylene/polypropylene. Alternatively, hollow fibers or fibers with an irregularly shaped cross-section may be used as the polyolefine-based fibers.

In addition to the polyolefine-based fibers, polyester-or polyamide-based fibers may be used as the synthetic fibers 8. It is also possible to blend at least two of the polyolefine-, polyester- and polyamide-based fibers as the synthetic fibers 8. As for the regenerated fibers 9, cellulose-based fibers such as rayon or acetate may be used.

The super-absorbent polymer particles 18 may be selected from a group of materials including a hydrolyzed compound of starch-acrylic acid graft copolymer, starch-based water absorbent resin obtained by neutralizing starch-acrylic acid graft copolymer, saponified vinyl acetate-acrylic ester copolymer, partially neutralized polyacrylic acid, and a polymer obtained from maleic anhydride-isobutylene copolymer and water-soluble ethylene-based unsaturated monomer. The super-absorbent polymer particles 18 may be incorporated into both the synthetic fibers 8 and the regenerated fibers 9.

The fibrous assembly 5 is provided for the purpose of protecting the wearer from having an uncomfortable feeling when the wearer's skin is touched with the absorbent material 4 and serves also as a cushion. The fibrous assembly 5 is a mixture of the synthetic fibers, and the regenerated fibers and fluff pulp, which are compressed together to a desired thickness.

Content of the super-absorbent polymer particles 1B in the yarns 7a is preferably in a range of 30 - 60 wt%. If the content of the super-absorbent polymer particles 18 is less than 30 wt%, a body fluid absorbing capacity of the absorbent material 4 will be decreased. With the yarn 7a wherein the synthetic fibers 8 contains within itself the super-absorbent polymer particles 18, the content of the super-absorbent polymer particles 18 exceeding 60 wt% would reduce the strength of the fibers 8 when the fibers 8 is wetted, so such anxiety is raised that the fibers 8 itself can not keep its shape.

In the absorbent material 4, fineness of the synthetic fibers 8 and the regenerated fibers 9 is preferably in a range of 1.5 - 100 dtex. If the fineness of these fibers 8, 9 is less than 1.5 dtex, strength of these fibers 8, 9 would be decreased and easily damaged. If the fineness of these fibers 8, 9 exceeding 100 dtex, on the contrary, stiffness of the yarns 7a would be raised too high to keep desirable flexibility of the absorbent material 4.

The number of individual fibers 8, 9 in the yarns 7a is preferably in a range of 20 - 800. If the number of the individual fibers 8, 9 is less than 20, the body fluid absorbing capacity of the absorbent material 4 would be decreased and flexural modulus as well as flexural recovering property would be also reduced. If the number of the individual fibers 8, 9 exceeds 800, diameter of the yarns 7a would be excessively increased and consequently thickness of the absorbent material 4 would be unacceptably increased.

Along the longitudinally opposite ends 1a, ribbon-like elastic members 10 associated with a waist-hole extend in the transverse direction and are secured in a stretched state to the diaper 1. In the crotch region 16, the transversely opposite side edges 1b have elastic members 11 associated with leg-holes extending in the longitudinal direction, each of these elastic members 11 comprising a plurality of elastic elements and secured in a stretched state to the diaper 1.

The leak-barrier cuffs 6 extend across the crotch region 16 into the front and rear waist regions 15, 17 in the vicinity of the respective side edges 1b. Each of the cuffs 6 has a fixed side edge portion 6a extending across the crotch region 16 in the longitudinal direction, a free side edge portion 6b normally biased to rise on the topsheet 2 and longitudinally opposite fixed end portions 6c lying in the front and rear waist regions 15, 17, respectively, and collapsed inwardly in the transverse direction of the diaper 1. Of the cuffs 6, the fixed side edge portions 6a are joined to the transversely opposite side edges 1b, respectively, and the longitudinally opposite fixed end portions 6c are joined to the longitudinally opposite ends 1a, respectively.

Each of the cuffs 6 further includes a lateral portion 6d extending outwardly from the fixed side edge portion 6a in the transverse direction. The free side edge portion 6b has an elastic member 12 extending in the longitudinal direction and secured in a stretched state thereto. This elastic member 12 is wrapped with a part of the free side edge portion 6b.

As the diaper 1 is curved in the longitudinal direction with the topsheet 2 inside, contraction of the respective elastic members 12 causes the respective free side edges 6b of the cuffs 6 to be contracted and thereby to rise on the topsheet 2. In the diaper 1, the free side edge portions 6b of the cuffs 6 thus rising on the topsheet 2 form barriers against body fluids and prevent body fluids from leaking from the crotch region 16.

Of the diaper 1, the transversely opposite side edges 1b of the rear waist region 17 are provided with a pair of tape fasteners 13, respectively, and the backsheet 3 in the front waist region 15 is provided on its outer surface with a rectangular target tape strip 14 functioning as a landing zone for the tape fasteners 13.

Each of the tape fasteners 13 is made of a flexible plastic film and has a proximal end portion attached to the associated side edge 1b of the rear waist region and a free end portion adapted to be temporarily anchored on the target tape strip 14. Of the tape fastener 13, the free end portion is coated with self adhesive (not shown) so as to be temporarily fixed to the associated cuff 6 by means of adhesive. The target tape strip 14 is made of a flexible plastic film.

End portions 2a of the topsheet 2 as well as end portions 3a of the backsheet 3 extend outwardly beyond the opposite ends 1a of the absorbent material 4 in the longitudinal direction and these ends 2a, 3a are overlapped and joined to each other. The longitudinally opposite fixed end portions 6c of the respective cuffs 6 are joined to the respective end portions 2a of the topsheet 2. The elastic members 10 associated with the waist-hole are interposed between the end portions Za of the topsheet 2 and the end portions 3a of the backsheet 3 and secured to these end portions 2a, 3a.

In the vicinity of the transversely opposite side edges 1b, the opposite side edge portions 2b of the topsheet 2 extend slightly outward of the opposite side edges of the absorbent material 4, and the opposite side edge portions 3b of the backsheet 3 and the lateral portions 6d of the respective cuffs 6 extend outwardly beyond the opposite side edge portions 2b of the topsheet 2. The side edge portions 2b and the side edge portions 3b overlap and are joined to each other, respectively. The side edge portions 3b and the lateral portions 6d overlap and are joined to each other, respectively. The fixed side edge portions 6a of the respective cuffs 6 are joined to the associated side edge portions 2b of the topsheet 2. The elastic members 11 associated with the leg-holes are interposed between the side edge portions 3b of the backsheet 3 and the lateral portions 6d of the cuffs 6 and secured to these side edge portions 3b and lateral portions 6d, respectively.

To wear the diaper 1, the opposite side edges 1b of the rear waist region 17 may be placed upon the opposite side edges 1b of the front waist region 15 and the free end portions of the tape fasteners 13 may be anchored on the target tape strip 14 by means of adhesive. With the front and rear waist regions 15, 17 connected to each other in this manner, the diaper 1 defines the waist-hole and the pair of leg-holes.

Of the diaper 1, the absorbent material 4 is provided in a form of woven fabric obtained from the yarns 7a which serves to restrict relative movement of the yarns in the absorbent material 4 and thereby to prevent the shape of the absorbent material 4 from being lost. In other words, other members are not necessarily required to keep the shape of the absorbent material 4. Even when the diaper 1 is bent or twisted, the absorbent material 4 is freely deformed following such bending or twisting, so there is no anxiety that the absorbent material 4 might restrict movement of the diaper 1.

In the diaper 1, body fluids permeated through the topsheet 2 and the fibrous assembly 5 can be reliably absorbed and retained by the super-absorbent polymer particles 18 so that back flow of body fluids to the topsheet 2 as well as sticking of body fluids to the wearer's skin can be completely avoided.

While the diaper 1 has been illustrated and described as having the fibrous assembly 5 placed on the upper surface of the absorbent material 4, the fibrous assembly 5 is not essential and it is also possible to interpose nothing but the absorbent material 4 between the topsheet 2 and the backsheet 3. Furthermore, an arrangement is also possible in which the diaper 1 is provided with more than two absorbent materials stacked in the thickness direction.

The topsheet 2 may be formed of a hydrophilic fibrous nonwoven fabric or a plastic film with a large number of micro-pores. The backsheet 3 may be formed of a hydrophobic fibrous nonwoven fabric, a liquid-impervious plastic film, two-layers of hydrophobic fibrous nonwoven fabric laminated with each other or a composite sheet composed of a hydrophobic fibrous nonwoven fabric and a plastic film joined to this hydrophobic fibrous nonwoven fabric. The leak-barrier cuffs 6 may be formed of a hydrophobic fibrous nonwoven fabric.

The backsheet 3 and the leak-barrier cuffs 6 may be also formed of a composite nonwoven fabric consisting of a highly water-resistant fibrous nonwoven fabric by melt blown method sandwiched by two-layers of fibrous nonwoven fabric having high strength and flexibility by spun bond method.

Nonwoven fabric used herein may be selected from a group of fabrics including those obtained by spun lace-, needle punch-, melt blown-, thermal bond-, spun bond-, chemical bond- and air through-processes. component fibers of such nonwoven fabric may be selected from a group of materials consisting of polyolefine-, polyester- and polyamide-based fibers and core-sheath type or side-by-side type conjugated fibers of polyethylene/polypropylene or polyethylene/polyester.

Joining of the top- and backsheets 2, 3 and the leak-barrier cuffs 6 as well as of the absorbent material 4 and the fibrous assembly 5, and attachment of the elastic members 10, 11, 12 may be carried out using a hot melt adhesive or welding technique such as heat-sealing or ultrasonic sealing.

The absorbent material 4 is applicable not only to the open-type diaper 1 and a sanitary napkin but also to a pants-type diaper with its front and rear waist regions previously connected to each other and a liquid-absorbent pad adapted to be attached to a diaper cover in actual use. In addition, the absorbent materials are applicable also to a blood staunching bandage, a sweat absorbent sheet and a mother's milk pad.

## Claims

1. A body fluid absorbent article (1) comprising:
an absorbent material(4) comprising a fabric made of yarns (7a) which are composed of synthetic fibers (8) obtained by spinning a melt thermoplastic synthetic resin blended with super-absorbent polymer particles (18) wherein, within the synthetic fibres, super-absorbent polymer particles are included, and, wherein said fabric is a net-like fabric woven by the yarns and said yarns further comprise regenerated fibers (9).

2. A body fluid absorbent article according to Claim 1, wherein a content of said super-absorbent polymer particles in said yarns is 30 - 60 wt%, a fineness of each of said synthetic fibers and said regenerated fibers is in a range of 1.5 - 100 dtex and the number of said synthetic fibers and said regenerated fibers in each of said yarns is in a range of 20 - 800.

3. A body fluid absorbent article according to any preceding claim, wherein said synthetic fibers are made of at least one of polyolefine-based fibers, polyester-based fibers, polyamide-based fibers, and said regenerated fibers are made of cellulose- based fibers.

4. A body fluid absorbent article according to any preceding claim, wherein said absorbent material is interposed between a liquid-pervious topsheet (2,21) facing a wearer's skin and a liquid-impervious backsheet(3,22)facing away from the wearer's skin.

## Patentansprüche

1. Absorbierender Artikel für Körperflüssigkeiten (1), der Folgendes umfasst:
ein absorbierendes Material (4), das ein Gewebe aus Garnen (7a) umfasst, die aus synthetischen Fasern (8) bestehen und die gewonnen werden, indem ein geschmolzener thermoplastischer synthetischer Harz mit superabsorbierenden Polymerteilchen (18) gesponnen wird, wobei innerhalb der synthetischen Fasern superabsorbierende Polymerteilchen beinhaltet sind, und worin das Gewebe ein genetztes Gewebe ist, das aus den Garnen gewebt wurde, und die Garne ferner regenerierte Fasern (9) umfassen.

2. Absorbierender Artikel für Körperflüssigkeiten nach Anspruch 1, wobei ein Inhalt der superabsorbierenden Polymerteilchen in den Garnen 30 - 60 Gew.% ist, eine Feinheit der synthetischen Fasern und der regenerierten Fasern in einem Bereich von 1,5 - 100 dtex ist und die Anzahl der synthetischen Fasern und regenerierten Fasern in jedem der Garne im Bereich von 20 - 800 ist.

3. Absorbierender Artikel für Körperflüssigkeiten nach einem vorangehenden Anspruch, wobei die synthetischen Fasern aus mindestens einem auf Polyolefine basierenden Fasern, auf Polyester basierenden Fasern und auf Polyamid basierenden Fasern gemacht sind, und die regenerierten Fasern aus auf Zellulose basierenden Fasern gemacht sind.

4. Absorbierender Artikel für Körperflüssigkeiten nach einem vorangehenden Anspruch, wobei das absorbierende Material zwischen einer flüssigkeitsdurchlässigen oberen Schicht (2, 21), die der Haut des Trägers zugewandt ist, und einer flüssigkeitsundurchlässigen hinteren Schicht (3, 22) liegt, die von der Haut des Trägers abgewandt ist.

## Revendications

1. Article absorbant les fluides corporels (1) comportant :
un matériau absorbant (4) comportant un tissu réalisé à partir de fils (7a) qui sont composés de fibres synthétiques (8) obtenues par le filage d'une résine synthétique thermoplastique à l'état fondu mélangée avec des particules de polymère super absorbantes (18) dans lequel, à l'intérieur des fibres synthétiques, des particules de polymère super absorbantes sont incluses, et, dans lequel ledit tissu est un tissu similaire à un filet tissé par les fils et lesdits fils comportent par ailleurs des fibres régénérées (9).

2. Article absorbant les fluides corporels selon la revendication 1, dans lequel une teneur desdites particules de polymère super absorbantes dans lesdits fils est de 30 à 60 en % en poids, une finesse de chacune desdites fibres synthétiques et desdites fibres régénérées est de l'ordre de 1,5 à 100 dtex et le nombre de dites fibres synthétiques et de dites fibres régénérées dans chacun desdits fils est de l'ordre de 20 à 800.

3. Article absorbant les fluides corporels selon l'une quelconque des revendications précédentes, dans lequel lesdites fibres synthétiques sont réalisées à partir d'au moins l'une parmi des fibres à base de polyoléfine, des fibres à base de polyester, des fibres à base de polyamide, et lesdites fibres régénérées sont réalisées à partir de fibres à base de cellulose.

4. Article absorbant les fluides corporels selon l'une quelconque des revendications précédentes, dans lequel ledit matériau absorbant est intercalé entre une feuille de dessus perméable aux liquides (2, 21) orientée vers la peau d'un utilisateur et une feuille de support imperméable aux liquides (3, 22) orientée à l'opposé par rapport à la peau de l'utilisateur.
